# EUROPEAN PATENT SPECIFICATION

(11) **EP 0 972 537 B1**
(45) Date of publication and mention of the grant of the patent: **18.05.2005**
(21) Application number: 99304724.0
(22) Date of filing: 17.06.1999
(51) Int. Cl.: A61M 25/02

(54) **Medical fixation device**
Medizinische Fixierungsvorrichtung
Dispositif de fixation médical

(30) Priority: 18.06.1998 GB 9813056
(43) Date of publication of application: 19.01.2000
(73) Proprietor: Mogg, Alan David, Ferndown, Dorset BH22 9HE (GB)
(72) Inventor: Mogg, Alan David, Ferndown, Dorset BH22 9HE (GB)
(74) Representative: Lomas, Geoffrey Michael

(56) References cited:
- EP-A- 0 807 450
- DE-A- 4 434 262
- US-A- 3 702 612
- US-A- 3 782 388
- US-A- 3 794 032
- US-A- 4 606 735
- US-A- 4 769 010
- US-A- 5 137 519
- US-A- 5 188 609
- US-A- 5 352 211

## Description

This invention relates to a fixation device for the attachment of rigid or semi-rigid medical items of medical equipment to the skin of a patient.

In many medical treatments, there is a requirement to use items of medical equipment which, either through convenience or necessity, have to be attached to a patient's skin. One example of such equipment comprises a biological filter used with an epidural catheter, where the filter has to be attached to the patient in order to avoid the weight of the filter pulling the catheter from the patient.

Many of the medical items which require attachment, such as a biological filter, are rigid and hard and of a size that cannot be comfortably attached to the patient's skin. The general practice in this situation is for the medical device to be mounted on a soft pad of material, and then fixed with adhesive tape to the patient. These ad hoc methods of attachment are uncomfortable and unsightly and do not give very secure or consistent results.

Fixing rigid or semi-rigid items to the skin is difficult, because of the irregular, undulating and dynamic nature of the body's skin surface. Fixing these items directly to the skin, even by means of a thin sticky pad, can cause discomfort and/or restrict the free movement of the patient around the fixation site. Movement of the patient can also cause the means of attachment to become loose, by mechanical action working on the adhesive joint between the skin and device.

When attaching medical items to the skin three main issues affect the comfort of the patient. The first is the hardness or rigidity of the medical item. The second is the size of the medical item and the third being the natural characteristics of the patient's body at the site where the medical item is to be mounted.

The larger the medical item fixed to the skin the greater the restriction caused to the patient's body and the potential discomfort.

Also where the body has little fleshy cover but large amounts of joint articulation, muscle and tendon movement etc. then the attachment of the medical item also causes greater restriction and discomfort.

US 4606735 discloses a catheter holder which holds and immobilises a catheter above the skin and the holder being secured to the skin by way of a fabric hook and loop fastener, one part of the fastener being secured to the skin (which part is not a pad of resilient foam plastics material). The main objective of the holder of US 4606735 is to immobilise the catheter.

US 3702612 discloses a catheter support comprising a resilient post the uppermost end of which is provided with a catheter receiving formation and at the lowermost end is supported on a pedestal, the pedestal being provided on a base plate and the base plate being provided with an adhesive layer for attachment to the skin. In use flexure is achieved by the resilience of the post and not by the pedestal.

US 4769010 discloses a catheter needle support assembly.

US 5352211 discloses a flexible stability device for guiding a conduit into a patient's skin.

For the comfortable attachment of some semi-rigid or rigid medical items I consider that an attachment method requires a flexible element to dynamically isolate the patients skin from the medical item, the degree of this flexibility being dependant on the rigidity and size of the medical item and the position it is to be mounted on the patient.

The biological filter used with an epidural catheter for example is made of a rigid material which can be 60 mm in diameter, and which is usually positioned on the front part of the patients shoulder where the body has high levels of internal and skin activity. In this situation I consider that a flexible mounting method is necessary to allow the patient full movement without discomfort.

According to the invention a fixation device for attachment of a rigid or semi-rigid item of medical equipment to the skin of a patient comprises a skin mounting means adapted to be secured to the skin in face contact therewith or by means of an adhesive layer, an item support structure carried by the skin mounting means and adapted to be secured to the medical equipment item, wherein the skin mounting means is connected to the item support structure by an upstanding spacer structure that defines, in use, a space between the skin on the one hand, and the item with item support structure on the other hand, and characterised in that the skin mounting means comprises a pad of resilient foam plastics material on which there is provided a flexible membrane of plastics material, the item spacer structure being attached to the membrane, and the flexible membrane is arranged to flex relative to the spacer structure on flexure of the skin relative to the item.

Thus the item support structure and the item itself are held clear of the skin by the upstanding spacer structure, and a degree of flexibility is provided in the membrane, to enable local flexing of the skin relative to the item, to accommodate body movement.

In one preferred embodiment the item support structure is integral with the item.

In other preferred embodiments, the spacer structure comprises a plurality of laterally spaced-apart support pillars.

In order to permit said flexing of the skin, the support pillars may be of resilient material.

Alternatively, the support pillars are relatively rigid and the flexible membrane provides a respective foot carried by the lower end of the respective pillar.

Advantageously, each foot then comprises a plurality of webs.

The skin mounting means may be adapted to be attached to the skin of a patient by sutures.

Alternatively, the skin mounting means carries self-adhesive means for attaching it to the skin of a patient.

The minimum distance in use between the item support structure and an item, and flat areas of skin on which the device is mounted, is preferably substantially in the range 5 to 10 mm.

When the item is circular in plan, said minimum distance is typically substantially one sixth of the diameter of the item.

Various embodiments of the invention will now be described by way of example only with reference to the accompanying drawings, wherein:-
**Figure 1** is a view in perspective of a first fixation device, supporting a biological filter,
**Figure 2** is a plan view thereof, with the biological filter removed,
**Figure 3** is a view in section, taken on the lines E-E of Figure 2,
**Figure 4** is a side view of a complete fixation device, mounted on the skin of a patient,
**Figure 5** is a view in section, taken on the lines F-F of Figure 4,
**Figure 6** is a fragmentary side view, in section, taken on the lines D-D of Figure 2, with the device mounted on the skin of a patient,
**Figure 7** is a fragmentary side view, in section, of a first modification of a device, mounted on the skin of a patient,
**Figure 8** is a plan view, in section, taken on the lines G-G of Figure7,
**Figure 9** is a fragmentary side view, partly in section, of a modification, with the device mounted on a patient's skin,
**Figure 10** is a plan view, taken on the lines J-J of Figure 9,
**Figure 11** is a side view of a second fixation device, mounted on the skin of a patient,
**Figure 12** is a plan view, in section, taken on the lines L-L of Figure 11,
**Figure 13** is a side view of a third form of fixation device, mounted on the skin of a patient, and
**Figure 14** is a plan view thereof looking in the direction of arrow M.

In the Figures, like features and components have like reference numerals.

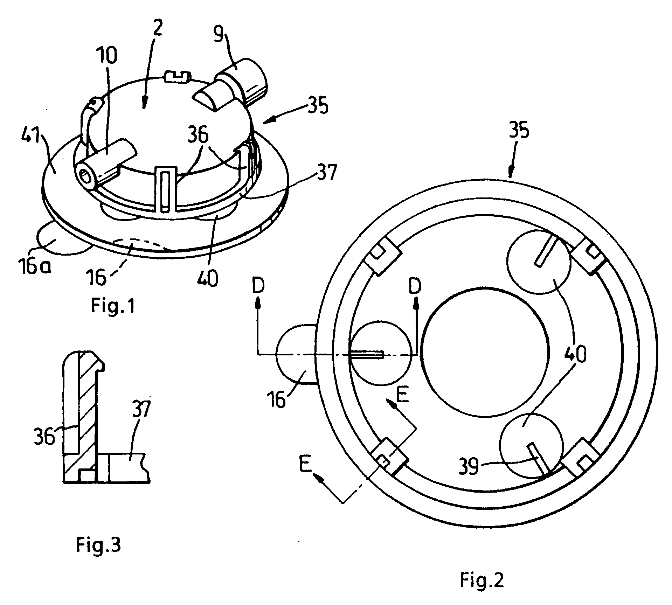

In the device 35 of Figures 1, 2 and 3, a rigid item 2 of medical equipment, which comprises a biological filter, having flow ducts 9 and10 is releasably held in place by an annular cage-like structure 36 of plastics material, having equi-spaced, finger-like spring clips 36 which resiliently secure the filter 2 in place on a support ring 37.

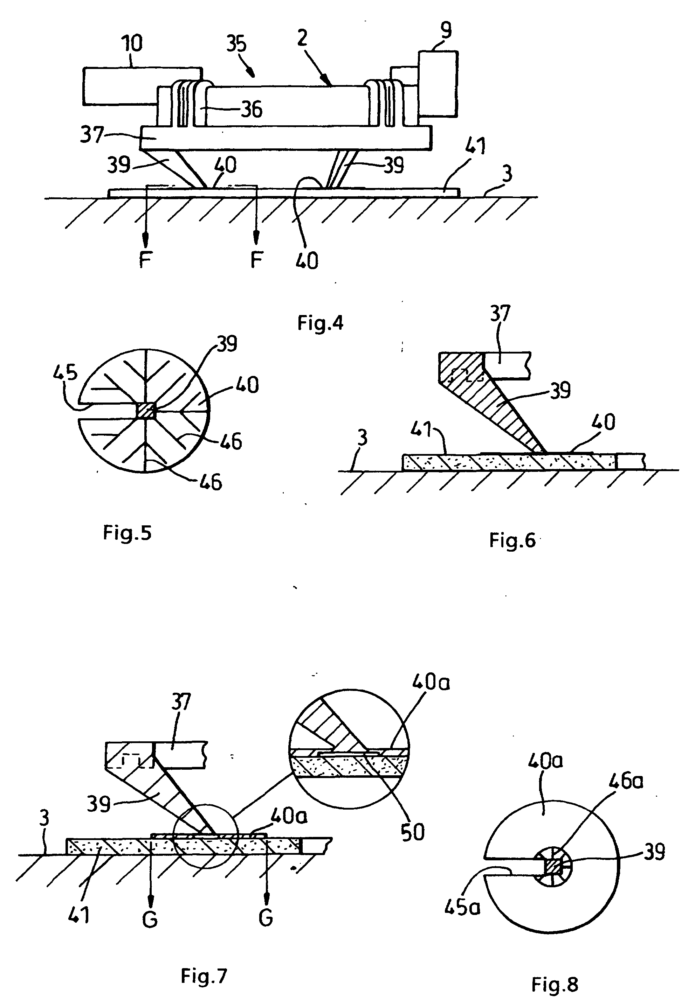

The ring 37 is flexibly supported above a body comprising a pad 41 of resilient foam plastics material, by way of three equi-spaced, legs or pillars 39 (Figure 6), of plastics material. Release paper 16 with a release tab 16a is provided to prevent adhesion until the fixation device 35 is ready for mounting on the skin The pillars 39 extend downwardly between the support ring 37 and thin (eg 0.2 mm) membranes 40 of plastics material disposed on the upper surface of the pad 41, which is of resilient foam plastics material.

The membranes 40 and pillars 39 each comprise an integral moulding. As shown in Figure 4, the membranes 40, which provide support feet, each have slots 45 to assist moulding, and thin vein-like webs 46 distributed over the membranes so as to improve flow of the plastics material to the membranes, when moulding takes place.

Adhesive material is disposed between the under surfaces of the membranes 40 and the upper surface of the pad 41. Also between the lower surface of the pad 41 and the skin.

The pillars 39 and membranes or feet 40 are integral with the support ring 37.

When the device 35 is in use, mounted on the skin 3, the skin can move, without inflicting discomfort to the patient, relative to the item 2 and support structures 36, 37 and 39. The three legs or pillars 39 provide tripod-like support and the thin membranes or feet 40, being flexible, allow this relative movement. Additional flexibility is provided by the resilient pad 41.

With reference to Figures 7 and 8, feet or membranes 40a may be used, wherein their lower surfaces are formed with shallow (circular) recesses 50. The webs 46a shown in Figure 8 do not extend over such a large area as webs 46 of Figure 5, but are local to the associated pillars 39. A membrane 40a is preferably more rigid than a membrane 40 of Figure 5, but flexibility is provided by resilience of the webs 46a. The webs 46a, unlike webs 46, do not have plastic material between them.

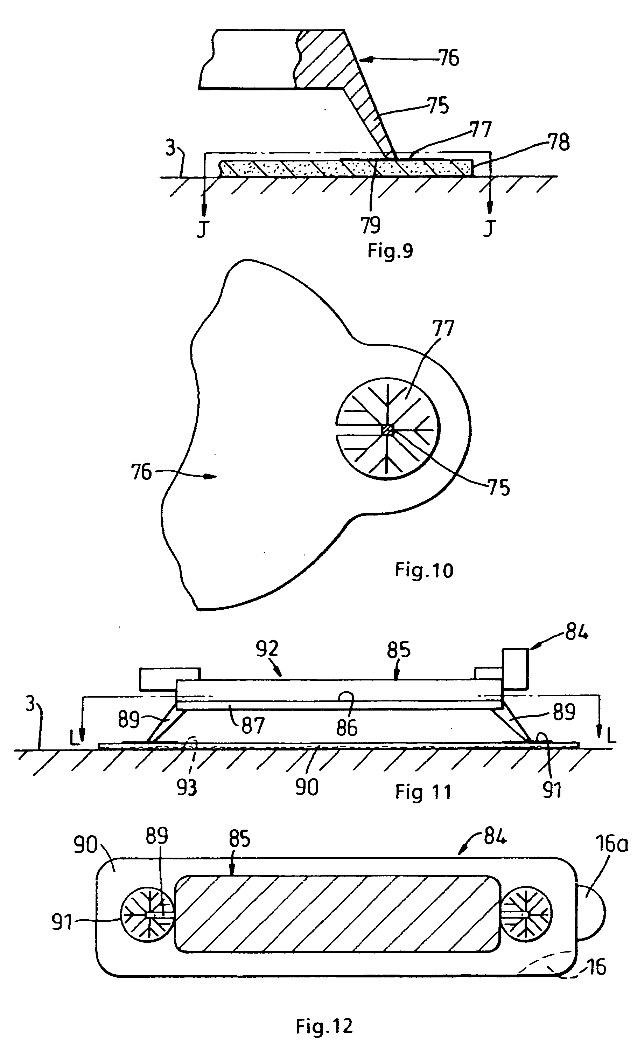

Figures 9 and 10 show a modification employing a spacer structure in the form of a plurality of support legs or struts 75 which are integral with a medical item 76, comprising a biological filter. The modification also makes use of thin membranes 77, substantially identical to membranes 40 of Figure 5, and integral with the struts 75. The membranes 77 are bonded to a pad 78 of foamed plastics material, by layers 79 of adhesive.

With reference to Figures 11 and 12, a fixation device 92 has an elongate biological filter 85, bonded at 86 to a filter support structure 87 supported by a pair of integral struts 89, above a pad 90 of foamed plastics material. The struts 89 employ integral membranes 91, substantially identical to membranes 40 of Figure 5.

The device 92 is secured to the skin 3 of patient by a layer 93 of self-adhesive. A release tag 16a is provided, so as to allow removal of release paper (not shown), prior to application of the device 92 to the patient's skin 3.

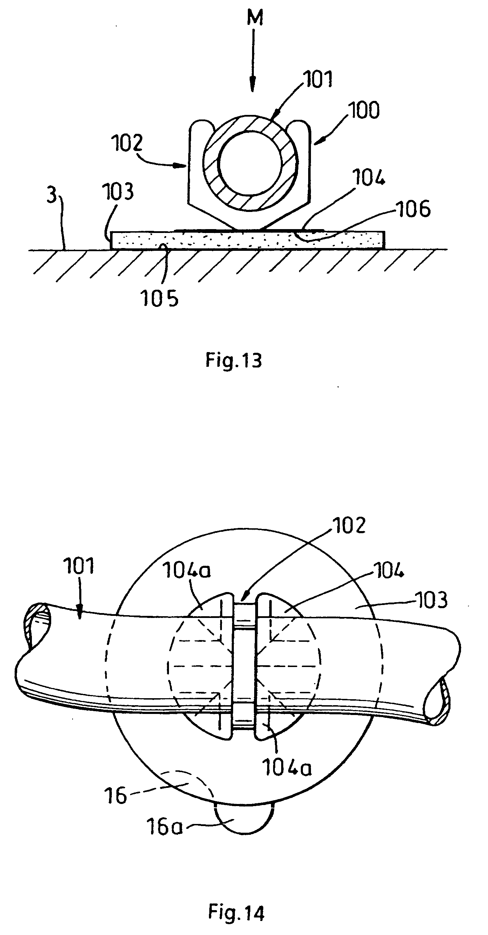

Figures 13 and 14 together illustrate a fixation device 100 for attachment of an item 101 of medical equipment, namely a flexible tube, to the skin 3 of a patient. The device 101 employs support structure in the form of a U-shaped spring clip 102, resiliently attached to a pad 103 of foamed plastics material by a two-piece membrane 104, each piece 104a of the membrane 104 being of substantially semi-circular form.

The membrane 104 and spring clip 102 comprise an integral component of plastics material. The pad 103 is secured to the skin 3 by a layer 105 of self-adhesive and the membrane 104 is bonded to the pad 103 by a layer 106 of adhesive. A release tag 16a is provided, so as to permit removal of release paper (not shown) prior to application of the device 100 to the patient's skin.

Where practicable and where desirable, features disclosed herein may be substituted for, or added to other such features.

Thus, for example, where a device has been described as attachable to a patient's skin by means of sutures, it may instead be attachable by self-adhesive material, or a combination of sutures and such material.

## Claims

1. A fixation device (35; 92; 100) for attachment of a rigid or semi-rigid item (2; 76; 85; 101) of medical equipment to the skin of a patient comprising a skin mounting means adapted to be secured to the skin in face contact therewith or by means of an adhesive layer, an item support structure (36; 37; 87; 102) carried by the skin mounting means and being secured to or adapted to be secured to the medical equipment item, wherein the skin mounting means (41; 78; 90; 103) is connected to the item support structure by an upstanding spacer structure (39; 75; 89) that defines, in use, a space between the skin (3) on the one hand and the item (2; 76; 85; 101) with item support structure (36; 37; 102) on the other hand, and **characterised in that** the skin mounting means comprises a pad of resilient foam plastics material on which there is provided a flexible membrane of plastics material (40; 40a; 77; 91; 104), the item spacer structure being attached to the membrane, and the flexible membrane is arranged to flex relative to the spacer structure on flexure of the skin relative to the item.

2. A device as claimed in claim 1, **characterised in that** the item support structure (36; 37; 87; 102) is integral with the item (76).

3. A device as claimed in claim 1 or claim 2 **characterised in that** the spacer structure comprises a plurality of laterally spaced-apart support pillars (39; 75; 89).

4. A device as claimed in claim 3, **characterised in that** the support pillars (39; 75; 89) are of resilient material.

5. A device as claimed in claim 3, **characterised in that** the support pillars (39; 75; 89) are relatively rigid and the flexible membrane (40; 40a; 77; 91; 104) provides a respective foot carried by the lower end of the respective pillar.

6. A device as claimed in claim 5, **characterised in that** each foot comprises a plurality of webs (46, 46a).

7. A device as claimed in any one of claims 3 to 6, wherein the item support structure (36; 37; 87; 102) and its support pillars comprise an integral moulding of plastics material.

8. A device as claimed in any one of the preceding claims, wherein the item (101) comprises a tube of plastics material.

9. A device as claimed in any of the preceding claims **characterised in that** the skin mounting means (41; 78; 90; 103) is adapted to be attached to the skin (3) of a patient by sutures.

10. A device as claimed in any of claims 1 to 11 **characterised in that** the skin mounting means (41; 78; 90; 103) carries self-adhesive means (93; 105) for attaching the skin mounting means to the skin of a patient.

11. A device as claimed in any of the preceding claims **characterised in that** the minimum distance in use between the item support structure (36; 37; 87; 102) and item (2; 76; 85; 101), and a flat area of skin on which the device is mounted, is substantially in the range 5 to 10 mm.

## Patentansprüche

1. Befestigungsvorrichtung (35; 92; 100) zum Befestigen eines steifen oder halbsteifen Gegenstandes (2; 76; 85; 101) einer medizinischen Ausstattung an der Haut eines Patienten, aufweisend ein Haut-Befestigungsmittel, das angepasst ist, an der Haut in Flächenkontakt damit oder mittels einer Haftschicht befestigt zu werden, eine Gegenstand-Haltestruktur (36; 37; 87; 102), welche durch das Haut-Befestigungsmittel getragen wird und welche befestigt wird mit oder angepasst wird befestigt zu werden an dem medizinischen Ausstattungsgegenstand, wobei das Haut-Befestigungsmittel (41; 78; 90; 103) mit der Gegenstand-Haltestruktur durch eine aufrechte Abstandstruktur (39; 75; 89) verbunden ist, welche in Verwendung einen Raum zwischen der Haut (3) einerseits und dem Gegenstand (2; 76; 85; 101) mit der Gegenstand-Haltestruktur (36; 37; 102) andererseits definiert und **dadurch gekennzeichnet, dass** die Haut-Befestigungsmittel ein Polster aus elastischem Schaumkunststoffmaterial aufweisen, an welchem eine flexible Membran aus Kunststoffmaterial (40; 40a; 77; 91; 104) vorgesehen ist, wobei die Gegenstand-Abstandstruktur an der Membran befestigt ist und die flexible Membran angeordnet ist, relativ zu der Abstandstruktur auf ein Biegen der Haut hin relativ zum Gegenstand zu biegen.

2. Vorrichtung nach Anspruch 1, **dadurch gekennzeichnet, dass** die Gegenstand-Haltestruktur (36; 37; 87; 102) integral mit dem Gegenstand (76) ist.

3. Vorrichtung nach Anspruch 1 oder 2, **dadurch gekennzeichnet, dass** die Abstandstruktur eine Mehrzahl von seitlich beabstandeten Halterungsstützen (39; 75; 89) aufweist.

4. Vorrichtung nach Anspruch 3, **dadurch gekennzeichnet, dass** die Halterungsstützen (39; 75; 89) aus elastischem Material sind.

5. Vorrichtung nach Anspruch 3, **dadurch gekennzeichnet, dass** die Halterungsstützen (39; 75; 89) relativ steif sind und die flexible Membran (40; 40a; 77; 91; 104) einen jeweiligen Fuß bereitstellt, der durch das untere Ende der jeweiligen Stütze getragen wird.

6. Vorrichtung nach Anspruch 5, **dadurch gekennzeichnet, dass** jeder Fuß eine Mehrzahl von Rippen (46, 46a) aufweist.

7. Vorrichtung nach irgendeinem der Ansprüche 3 bis 6, wobei die Gegenstand-Haltestruktur (36; 37; 87; 102) und ihre Halterungsstützen eine integrale Ausformung aus Kunststoffmaterial aufweisen.

8. Vorrichtung nach irgendeinem der vorhergehenden Ansprüche, wobei der Gegenstand (101) eine Röhre aus Kunststoffmaterial aufweist.

9. Vorrichtung nach irgendeinem der vorhergehenden Ansprüche, **dadurch gekennzeichnet, dass** das Haut-Befestigungsmittel (41; 78; 90; 103) angepasst ist, an der Haut (3) eines Patienten durch Nähte befestigt zu werden.

10. Vorrichtung nach irgendeinem der Ansprüche 1 bis 9, **dadurch gekennzeichnet, dass** die Haut-Befestigungsmittel (41; 78; 90; 103) selbsthaftende Mittel (93; 105) tragen zum Befestigen der Haut-Befestigungsmittel an der Haut eines Patienten.

11. Vorrichtung nach irgendeinem der vorhergehenden Ansprüche, **dadurch gekennzeichnet, dass** der minimale Abstand in Verwendung zwischen der Gegenstand-Haltestruktur (36; 37; 87; 102) und dem Gegenstand (2; 76; 85; 101) und ein flacher Bereich von Haut, auf welchem die Vorrichtung befestigt ist, im Wesentlichen im Bereich von 5 bis 10 mm liegt.

## Revendications

1. Un dispositif de fixation (35 ; 92 ; 100) pour attacher un article rigide ou semi-rigide (2 ; 76 ; 85 ; 101) d'équipement médical à la peau d'un patient, comprenant des moyens de montage sur la peau aptes à être fixés à la peau en contact frontal avec celle-ci ou au moyen d'une couche adhésive, une structure (36 ; 37 ; 87 ; 102) de support d'article portée par les moyens de montage sur la peau et étant fixée ou apte à être fixée à l'article d'équipement médical, les moyens (41 ; 78 ; 90 ; 103) de montage sur la peau étant reliés à la structure de support d'article par une structure (39 ; 75 ; 89) d'espacement vertical qui définit, en service, un espace entre la peau (3) d'une part et l'article (2 ; 76 ; 85 ; 101) avec la structure (36 ; 37 ; 102) de support d'article d'autre part, et **caractérisé en ce que** les moyens de montage sur la peau comprennent un patin de matière plastique en mousse élastique, sur lequel est prévue une membrane flexible (40 ; 40a ; 77 ; 91 ; 104) en matière plastique, la structure d'espacement d'article étant fixée à la membrane, et **en ce que** la membrane flexible est agencée pour fléchir par rapport à la structure d'espacement en cas de flexion de la peau par rapport à l'article.

2. Un dispositif selon la revendication 1, **caractérisé en ce que** la structure (36 ; 37 ; 87 ; 102) de support d'article est monobloc avec l'article (76).

3. Un dispositif selon la revendication 1 ou la revendication 2, **caractérisé en ce que** la structure d'espacement comprend une pluralité de piliers de support (39 ; 75 ; 89) espacés les uns des autres.

4. Un dispositif selon la revendication 3, **caractérisé en ce que** les piliers de support (39 ; 75 ; 89) sont en matériau élastique.

5. Un dispositif selon la revendication 3, **caractérisé en ce que** les piliers de support (39 ; 75 ; 89) sont relativement rigides et **en ce que** la membrane flexible (40 ; 40a ; 77 ; 91 ; 104) fournit un pied respectif porté par l'extrémité inférieure du pilier respectif.

6. Un dispositif selon la revendication 5, **caractérisé en ce que** chaque pied comprend une pluralité de nervures (46, 46a).

7. Un dispositif selon l'une quelconque des revendications 3 à 6, dans lequel la structure (36 ; 37 ; 87 ; 102) de support d'article et ses piliers de support comprennent un moulage monobloc de matière plastique.

8. Un dispositif selon l'une quelconque des revendications précédentes, dans lequel l'article (101) comprend un tube en matière plastique.

9. Un dispositif selon l'une quelconque des revendications précédentes, **caractérisé en ce que** les moyens (41 ; 78 ; 90 ; 103) de montage sur la peau sont aptes à être fixés à la peau (3) d'un patient par des sutures.

10. Un dispositif selon l'une quelconque des revendications 1 à 11, **caractérisé en ce que** les moyens (41 ; 78 ; 90 ; 103) de montage sur la peau portent des moyens (93 ; 105) auto-adhésifs pour fixer les moyens de montage sur la peau à la peau d'un patient.

11. Un dispositif selon l'une quelconque des revendications précédentes, **caractérisé en ce que** la distance minimale en service entre la structure (36 ; 37 ; 87 ; 102) de support d'article et l'article (2 ; 76 ; 85 ; 101), et une surface plane de peau sur laquelle le dispositif est monté, est comprise sensiblement dans la plage de 5 à 10 mm.
